(19) Europäisches Patentamt
European Patent Office
Office européen des brevets

(11) Publication number: **0 021 475**
**B2**

# (12) NEW EUROPEAN PATENT SPECIFICATION

(45) Date of publication of the new patent specification: **22.01.86**

(51) Int. Cl.[4]: **C 01 B 33/28**

(21) Application number: **80200495.2**

(22) Date of filing: **23.05.80**

(54) **Crystalline silicates, process for their preparation and catalytic conversions using them.**

(30) Priority: **06.06.79 NL 7904432**

(43) Date of publication of application:
**07.01.81 Bulletin 81/01**

(45) Publication of the grant of the patent:
**14.09.83 Bulletin 83/37**

(45) Mention of the opposition decision:
**22.01.86 Bulletin 86/04**

(84) Designated contracting states:
**BE DE FR GB IT**

(56) References cited:
**BE-A- 871 893**
**DE-A- 2 446 012**
**DE-A- 2 755 770**
**DE-A- 2 818 307**
**DE-C- 1 467 175**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Boersma, Michael Adriaan Maria**
**Badhuisweg 3**
**NL-1031 CM Amsterdam (NL)**
Inventor: **Nanne, Johannes Martinus**
**Badhuisweg 3**
**NL-1031 CM Amsterdam (NL)**
Inventor: **Post, Martin Franciscus Maria**
**Badhuisweg 3**
**NL-1031 CM Amsterdam (NL)**
Inventor: **Schaper, Lambert**
**Badhuisweg 3**
**NL-1031 CM Amsterdam (NL)**

(74) Representative: **Aalbers, Onno et al**
**P.O. Box 302**
**NL-2501 CH The Hague (NL)**



Jouve, 18, rue St-Denis, 75001 Paris, France

**Description**

The invention relates to novel crystalline silicates as well as to a process for the preparation of these silicates. The invention further relates to the application of the silicates inter alia as adsorption and extraction agent, as desiccant, ion exchanger and as catalyst or catalyst carrier in the execution of a variety of catalytic processes, in particular the catalytic preparation of aromatic hydrocarbons from acyclic organic compounds.

From the German Patent Application 2,755,770 crystalline silicates are known having an X-ray powder diffraction pattern containing inter alia the reflections mentioned in the following Table A :

Table A

| Radiation : Cu-Kα<br>2θ | Wave length 0.15418 nm<br>relative intensity |
|---|---|
| 7.8-8.2 | S |
| 8.7-9.1 | S |
| 11.8-12.1 | W |
| 12.4-12.7 | W |
| 14.6-14.9 | W |
| 15.4-15.7 | W |
| 15.8-16.1 | W |
| 17.6-17.9 | W |
| 19.2-19.5 | W |
| 20.2-20.6 | W |
| 20.7-21.1 | W |
| 23.1-23.4 | VS |
| 23.8-24.1 | VS |
| 24.2-24.8 | M |
| 29.7-30.1 | M |

in which the letters used have the following significance :

VS = very strong ; S = strong ; M = moderate ; W = weak ; θ = the Bragg angle.

The silicates contain iron, and may contain aluminium and/or gallium. They may be used as catalysts and catalyst carriers.

Depending on the catalytic activity required, catalytically active metals can be deposited upon these silicates.

From the Belgian Patent BE-A 871, 893 it is known that catalytically active metals can be incorporated into the silicate structure.

The Applicant has found now that rhodium and/or scandium can be incorporated into the silicate structure. The new compounds prepared in this way show an excellent catalytic activity, especially in the preparation of aromatic hydrocarbons.

The invention, therefore, relates to catalytically active crystalline silicates having an X-ray powder diffraction pattern containing inter alia the reflections mentioned in Table A, characterized in that after calcination in air for one hour at 500 °C the composition expressed in moles of the oxides is as follows :

$$p(0.9 \pm 0.3)M_{2/n}O \cdot p(aX_2O_3 \cdot bY_2O_3) \cdot SiO_2$$

wherein

M = H and alkali metal and/or alkaline earth metal,

X = rhodium and/or scandium,

Y = aluminium, iron and/or gallium,

$a > 0.5$,

$b \geqslant 0$,

$a + b = 1$,

$0 < p < 0.1$ and

n = the valency of M.

Although the silicates according to the invention may in principle contain both elements X with or without two or three elements Y, preference is given for the above-mentioned applications to silicates containing only one element X with or without one element Y. Special preference is accorded to silicates which only contain one element X and no element Y, in other words, silicates with a general formula in which $b = 0$. As regards the presence of aluminium in the silicates according to the invention, the following should further be noted. The silicon compounds which are eligible from the economic angle for the preparation of crystalline silicates on a commercial scale usually contain a small quantity of

aluminium as impurity. At least part of this aluminium is usually found in the prepared silicate. This means that if it is envisaged to prepare crystalline silicates according to the invention which contain one or more elements X with or without iron and/or gallium, while starting from a basic mixture into which an aluminium-contaminated silicon compound is incorporated, a crystalline silicate according to the invention will as a rule be obtained which contains a small quantity of aluminium.

In the general formula of the silicates according to the invention p should have a value which is between 0 and 0.1. For the above-mentioned applications preference is given to silicates having a general formula in which p is larger than 0.001 and in particular larger than 0.002 5 as well as to silicates having a general formula in which p is smaller than 0.05.

The silicates according to the invention are defined inter alia with reference to the X-ray powder diffraction pattern which the silicate displays after calcination in air for one hour at 500 °C. This X-ray powder diffraction pattern should contain inter alia the reflections mentioned in Table A. The complete X-ray powder diffraction pattern of a typical example of a silicate according to the invention is represented in Table B (radiation : Cu-Kα ; wave length : 0.154 18 nm).

TABLE B

| $2\theta$ | Relative intensity ($100.I:I_0$) | Description |
|---|---|---|
| 8.00 | 55 | SP |
| 8.90 | 36 | SP |
| 9.10 | 20 | SR |
| 11.95 | 7 | NL |
| 12.55 | 3 | NL |
| 13.25 | 4 | NL |
| 13.95 | 10 | NL |
| 14.75 | 9 | BD |
| 15.55 | 7 | BD |
| 15.95 | 9 | BD |
| 17.75 | 5 | BD |
| 19.35 | 6 | NL |
| 20.40 | 9 | NL |
| 20.90 | 10 | NL |
| 21.80 | 4 | NL |
| 22.25 | 8 | NL |
| 23.25 | 100* | SP |
| 23.95 | 45 | SP |
| 24.40 | 27 | SP |
| 25.90 | 11 | BD |
| 26.70 | 9 | BD |
| 27.50 | 4 | NL |
| 29.30 | 7 | NL |
| 29.90 | 11 | BD |
| 31.25 | 2 | NL |
| 32.75 | 4 | NL |
| 34.40 | 4 | NL |
| 36.05 | 5 | BD |
| 37.50 | 4 | BD |
| 45.30 | 9 | BD |

*$I_0$ = intensity of the strongest discrete reflection which occurs in the pattern.

The letters used in Table B to describe the reflections have the following significance :
SP = sharp ; SR = shoulder ; NL = normal ; BD = broad. θ represents the Bragg angle.

The crystalline silicates according to the invention may be prepared starting from an aqueous mixture containing the following compounds : one or more compounds of an alkali or alkaline earth metal (M), one or more compounds containing an organic cation (R) or from which such a cation is formed during the preparation of the silicate, one or more silicon compounds, one or more compounds in which an element X occurs in the trivalent form and optionally one or more compounds in which aluminium or gallium occurs, or in which iron occurs in the trivalent form. The preparation occurs by maintaining the mixture, preferably with stirring, at elevated temperature until the silicate has been formed and by subsequently separating the silicate crystals from the mother liquor. In the aqueous mixture from which the silicates are prepared the various compounds should be present in the following ratio, expressed in moles of the oxides

$$M_{2/n}O : R_{2/q}O = 0.1\text{-}20,$$
$$R_{2/q}O : SiO_2 = 0.01\text{-}0.5,$$
$$SiO_2 : (X_2O_3 + Y_2O_3) > 10,$$
$$H_2O : SiO_2 = 5\text{-}50,$$

n is the valency of M and q is the valency of R.

In addition to novel silicates, the present patent application also relates to a process for the preparation of the novel silicates starting from an aqueous mixture having the above-mentioned composition by maintaining this mixture at elevated temperature until the silicate has been formed and by subsequently isomating the silicate from the mother liquor. The preparation of the silicates according to the invention may be carried out both at atmospheric pressure and at elevated pressure. If reaction temperatures are used which are above the boiling point of the mixture, use is preferably made of an autoclave at autogenous pressure. The silicates are preferably prepared by maintaining the mixture at a temperature between 90 and 300 °C and in particular at a temperature between 125 and 175 °C for at least 4 hours. After formation of the silicates the crystals are separated from the mother liquor, for example by filtration, decanting or centrifuging. The crystal mass is subsequently washed with water and finally dried at a temperature between 100 and 200 °C.

Examples of suitable compounds which may be used in the preparation of the silicates according to the invention are nitrates, carbonates, hydroxides and oxides of alkali and alkaline earth metals ; primary, secondary and tertiary alkyl amines and quaternary alkyl ammonium compounds, such as bromides and hydroxides ; heterocyclic amines such as pyridine and piperidine ; sodium silicate, silica gels, silicic acid, aqueous colloidal silica sols and amorphous solid silicas such as precipitated silica sols ; aluminium hydroxide, sodium aluminate and activated aluminas, such as gamma-alumina ; gallium oxide and gallium nitrate and compounds in which iron, scandium and rhodium occur in trivalent form, such as the oxides, the hydroxides, normal salts, in particular the nitrates and complex salts. In the preparation of the silicates according to the invention it is preferred to start from a basic mixture in which M is present in an alkali metal compound and R in tetra-alkyl ammonium compound and in particular from a basic mixture in which M is present in a sodium compound and R in a tetrapropyl ammonium compound.

Silicates prepared by heating the above-mentioned aqueous mixtures contain alkali and/or alkaline earth metal ions and organic cations. By using suitable exchange methods the alkali and alkaline earth metal ions can be substituted by other cations, such as hydrogen ions, ammonium ions or ions of the rare earth metals. Organic cations can very suitably be converted into hydrogen ions by calcining the silicates.

If the silicates according to the invention contain alkali metal and if it is envisaged to use the silicates as catalyst or catalyst carrier, it is preferred first to reduce the alkali metal content of these silicates to less than 1 %w and in particular to less than 0.05 %w. The reduction of the alkali metal content of the silicates can very suitably be carried out by contacting them once or several times with an aqueous solution which contains ammonium ions. From the $NH_4^+$ silicates obtained in this way the $H^+$ silicates can be prepared by calcination.

Silicates according to the invention possess catalytic activity and may therefore be used as such as catalysts. They may also be used as catalyst carriers for one or more catalytically active metal components. Very suitable catalysts for a great variety of processes are catalysts which contain one of the following metals or metal combinations supported on a silicate according to the invention as carrier : nickel, copper, zinc, cadmium, platinum, palladium, nickel-tungsten, cobalt-molybdenum, nickel-molybdenum, zinc-palladium, zinc-copper, and zinc-rhenium. Supporting the metals on the silicates may be effected in a conventional manner, for example by impregnation, percolation or ion exchange. In order to augment the activity, selectivity and/or stability of the catalysts, promoters may be incorporated such as halogen, magnesium, phosphorus, arsenic and antimony.

When using the silicates according to the invention for catalytic purposes these materials should as a rule be available in the form of particles having a diameter of 0.5-5 mm. In the mode of preparation as described above the silicates are obtained in the form of a fine powder. The silicates may be formed to particles having a larger dimension, for example by moulding. During moulding the silicates may be combined with a binder such as kaolin, bentonite and synthetic inorganic oxides. When the silicates according to the invention are combined with a binder any mixing ratio may in principle be used. Preference is given to binders which contain very little alkali metal, if any. For the sake of brevity catalysts which at least partly consist of a silicate according to the invention will further be designated as « catalysts according to the invention » in this patent application. When used for hydrocarbon conversion processes catalysts according to the invention as a rule display a higher stability according as the crystallite size of the silicate present in the catalyst is smaller. Mostly, silicates according to the invention are obtained with a smaller crystallite size according as a higher concentration of organic component is used in the basic mixture from which they are prepared.

Although the catalysts according to the invention display a longer life, they still have to be regenerated from time to time. This may be effected in a simple manner by burning.

Catalysts according to the invention may be used inter alia for the following processes :

1. The catalytic cracking of heavy hydrocarbon oils for the preparation of light hydrocarbon oil distillates ;

2. The preparation of isoparaffins by isomerization of n-paraffins ;
3. The hydrogenative desulphurization of hydrocarbon oil distillates ;
4. The conversion of naphthenes into aromatics ;
5. The polymerization of olefins for the preparation of polyolefins ;
6. The hydrocracking of heavy hydrocarbon oils for the preparation of light hydrocarbon oil distillates, such as the conversion of gas oil into gasoline ;
7. The hydrocracking of heavy hydrocarbon oils for the preparation of lubricating oils with a high viscosity index ;
8. The improvement of the light and oxidation stability of lubricating oils ;
9. The improvement of the octane number of gasoline ;
10. The preparation of olefins from lower alcohols and/or ethers ;
11. The preparation of olefinic gasoline having a low aromatics content from lower olefins or mixtures thereof with lower paraffins ;
12. The hydrogenative dewaxing of hydrocarbon oils such as lubricating oil and fuel for jet engines ;
13. The transalkylation of alkyl-substituted aromatics, such as the preparation of ethyl benzene from a mixture of benzene and diethyl benzene ;
14. The alkylation of aromatics, such as the preparation of ethyl benzene from benzene and ethylene.

Catalysts according to the invention are highly suitable for use in the following processes :

1. The catalytic dewaxing of gas oil to improve the cloud point ;
2. The preparation of p-xylene by isomerization of other $C_8$-aromatics ;
3. The preparation of p-xylene by methylation of toluene with, for example, methanol, methyl chloride or dimethyl ether ;
4. The preparation of p-xylene by disproportionation of toluene.

Although catalysts according to the invention may be successfully used for each of the above-mentioned processes, the great importance of these catalysts is nevertheless in another field, for it has been found that these catalysts are eminently suitable for the preparation of aromatic hydrocarbons from acyclic organic compounds. As starting material for this aromatics preparation many groups of organic compounds are eligible, such as alcohols, ethers, olefins, diolefins, paraffins, aldehydes, ketones and esters. It has been found that these catalysts not only have the property to form aromatics from organic compounds having 6 or more carbon atoms per molecule, such as hexadecene, but surprisingly are also capable of forming aromatics in a high yield from organic compounds having fewer than 6 carbon atoms per molecule, such as methanol, ethanol and propylene. Another surprising property of the catalysts according to the invention is that when they are used for the above-described preparation of aromatics, they yield a product in which the aromatics essentially contain at least 6 and at most 10 carbon atoms, irrespective of whether the preparation was started from organic compounds having 6 or more carbon atoms of from organic compounds having fewer than 6 carbon atoms. The latter property of the catalysts according to the invention is considered to be very important since aromatic compounds having 6-10 carbon atoms per molecule are eminently useful as gasoline components.

During the preparation of aromatics with the use of catalysts according to invention it is possible to start from both a certain acyclic, organic compound, such as methanol or propylene and of a mixture of mainly acyclic organic compounds. The aromatization process according to the invention is very suitable for the preparation of aromatics from methanol as well as for increasing the octane number of gasolines, such as straight-run gasolines and gasolines obtained during the hydro-, thermal and catalytic cracking of petroleum fractions.

The preparation of aromatic hydrocarbons from aliphatic and/or cycloaliphatic hydrocarbons is carried out by contacting the feed under aromatization conditions with a catalyst according to the invention. Examples of suitable starting materials for the preparation of aromatics are ethylene, propylene, butene, propane, butane, pentane, hexane, methyl pentane, methyl cyclopentane, Udex raffinates, straight-run gasoline fractions, pyrolysis gasoline fractions and products obtained in the Fischer-Tropsch hydrocarbon synthesis.

In addition to aliphatic and cycloaliphatic hydrocarbons, it is also possible to use as feed for the aromatization process according to the invention hydrocarbons which contain a hetero-atom such as an oxygen, halogen, sulphur or nitrogen atom. Examples of suitable compounds of this type are : methanol, ethanol, isopropanol, 2-ethyl hexanol, mixtures of oxo-alcohols, mixtures of pentanols, mixtures of methanol and propylene, methyl mercaptan, dimethyl ether, tri-n-butyl amine, methyl formiate, acetic acid, acetone, propion aldehyde, cyclopentanone, n-butyl formiate, n-propyl acetate and capronic acid.

The aromatization process according to the invention is particularly suitable for use with hydrocarbons and/or oxygen-containing hydrocarbons obtained in the conversion of synthesis gas, i. e. a gas mixture comprising carbon monoxide and hydrogen. The conversion of an $H_2/CO$ mixture into an aromatic hydrocarbon mixture with the use of a catalyst according to the invention may be carried out in one stage or in two stages. In the two-stage process an $H_2/CO$ mixture is contacted in the first stage with a catalyst which contains one or more metal components with catalytic activity for the conversion of an

$H_2/CO$ mixture into hydrocarbons and/or oxygen-containing hydrocarbons. In the second stage the resultant product is converted into an aromatic hydrocarbon mixture by contacting it under aromatization conditions with a catalyst according to the invention. In the one-stage process an $H_2/CO$ mixture is contacted with a bifunctional catalyst which, in addition to a crystalline silicate according to the invention contains one or more metal components with catalytic activity for the conversion of an $H_2/CO$ mixture into hydrocarbons and/or oxygen-containing hydrocarbons. The process is preferably carried out as a one-stage process. If the $H_2/CO$ mixture which is used as feed in the aromatization process according to the invention has an $H_2/CO$ molar ratio of less than 1.0 ($H_2/CO$ mixtures of this type are obtained inter alia by steam gasification of coal at elevated temperatures), the aromatization process is preferably carried out as a one-stage process by contacting the gas with a trifunctional catalyst which contains one or more metal components with catalytic activity for the conversion of an $H_2/CO$ mixture into hydrocarbons and/or oxygen-containing hydrocarbons, one or more metal components having catalytic activity for the water gas shift reaction and a crystalline silicate according to the invention. The ratio in which the three catalytic functions are present in the catalyst may vary within wide limits and is mainly determined by the activity of each of the catalytic functions. When a trifunctional catalyst is used in the aromatization process according to the invention for the conversion of an $H_2/CO$ mixture with an $H_2/CO$ molar ratio of less than 1.0, it is envisaged that the maximum quantity of the acyclic hydrocarbons and/or oxygen-containing hydrocarbons which have been formed under the effect of a first catalytic function is converted under the effect of a second catalytic function into an aromatic hydrocarbon mixture mainly boiling in the gasoline range, while at the same time the maximum amount of the water which is liberated in the conversion of the $H_2/CO$ mixture into hydrocarbons and/or in the conversion of oxygen-containing hydrocarbons into an aromatic hydrocarbon mixture reacts under the influence of a third catalytic function with the quantity of CO present in the $H_2/CO$ mixture in excess with formation of an $H_2/CO_2$ mixture.

Although the trifunctional catalysts which are used in the aromatization process according to the invention are described in this patent application as catalysts which contain one or more metal components with catalytic activity for the conversion of an $H_2/CO$ mixture into hydrocarbons and/or oxygen-containing hydrocarbons and one or more metal components with catalytic activity for the water gas shift reaction, this in no way means that separate metal components which each individually possess one of the two functions must invariably be present in the catalysts. For it has been found that metal components and combinations of metal components having catalytic activity for the conversion of an $H_2/CO$ mixture into mainly oxygen-containing hydrocarbons as a rule also have sufficient catalytic activity for the water gas shift reaction so that it will in that instance suffice to incorporate one metal component or one combination of metal components into the catalysts. Examples of such metal components are metals selected from the group formed by zinc, copper and chromium. When trifunctional catalysts which contain these metals are used in the aromatization process according to the invention, preference is accorded to catalysts which contain combinations of at least two of these metals, for example the combination zinc-copper, zinc-chromium or zinc-copper-chromium. Special preference is given to a trifunctional catalyst which contains the metal combination zinc-chromium in addition to the crystalline silicate. Metal components and combinations of metal components with catalytic activity for the conversion of an $H_2/CO$ mixture into mainly hydrocarbons possess as a rule insufficient activity, if any, for the water gas shift reaction. When such metal components are used in the catalysts, one or more separate metal components having catalytic activity for the water gas shift reaction must therefore be incorporated.

The trifunctional catalysts which are used in the aromatization process according to the invention are preferably composed of two or three separate catalysts which, for the sake of simplicity, will be designated as catalysts X, Y and Z. Catalyst X is the catalyst which contains the metal components with catalytic activity for the conversion of an $H_2/CO$ mixture into hydrocarbons and/or oxygen-containing hydrocarbons. Catalyst Y is the crystalline silicate. Catalyst Z is the catalyst which contains the metal components with catalytic activity for the water gas shift reaction. As has been explained above, the use of a catalyst Z may in some instances be omitted.

If as catalyst X a catalyst is used which is capable of converting an $H_2/CO$ mixture into mainly oxygen-containing hydrocarbons, a catalyst is preferred which is capable of converting the $H_2/CO$ mixture into mainly methanol and/or dimethyl ether. For the conversion of an $H_2/CO$ mixture into mainly methanol, catalysts containing the above-mentioned metal combinations are very suitable. If desired, the said metal combinations may be supported on a carrier. By incorporating an acid function into these catalysts, for example by supporting the metal combination on an acidic carrier, it is possible for the $H_2/CO$ mixture to be converted not only into methanol but also for a considerable part into dimethyl ether.

Catalysts X having activity for the conversion of an $H_2/CO$ mixture into mainly hydrocarbons are known as Fischer-Tropsch catalysts. As a rule these catalysts contain one or more metals of the iron group or ruthenium, together with one or more promoters to augment the activity and/or selectivity and sometimes a carrier material such as kieselguhr. If in the aromatization process according to the invention starting from an $H_2/CO$ mixture use is made of a catalyst combination in which catalyst X is a Fischer-Tropsch catalyst, preference is given to an iron or cobalt catalyst, in particular a catalyst of this type which has been prepared by impregnation. Examples of suitable Fischer-Tropsch catalysts are

catalysts prepared by impregnation which contain either iron, potassium and copper, or cobalt, thorium and magnesium, supported on silica as carrier. If desired, it is also possible to use in the aromatization process according to the invention starting from an $H_2/CO$ mixture catalyst combinations which contain a catalyst X capable of converting an $H_2/CO$ mixture into a mixture containing hydrocarbons and oxygen-containing hydrocarbons in comparable quantities. Such a catalyst also contains as a rule sufficient catalytic activity for the water gas shift reaction so that the use of a catalyst Z in the combination can be omitted. An example of a catalyst X of this type is an iron-chromium oxide catalyst. If desired, it is also possible to use in the aromatization process according to the invention starting from an $H_2CO$ mixture catalyst combinations which contain two or more catalysts of the type X, for example in addition to a first catalyst of the type X which is capable of converting an $H_2/CO$ mixture into mainly hydrocarbons a second catalyst of the type X which is capable of converting an $H_2/CO$ mixture into mainly oxygen-containing hydrocarbons.

Catalysts Z which are capable of converting an $H_2O/CO$ mixture into an $H_2/CO_2$ mixture are known in the literature as CO-shift catalysts. A very suitable catalyst Z for the present purpose is a catalyst containing zinc and copper.

The conversion of an $H_2/CO$ mixture into an aromatic hydrocarbon mixture according to the invention is preferably carried out at a temperature between 200 and 500 °C and in particular between 300 and 450 °C, a pressure between 1 and 150 bar and in particular between 5 and 100 bar and a liquid hourly space velocity of 50-5 000 and in particular 300-3 000 Nl of gas/l of catalyst/hour. In the preparation of an aromatic hydrocarbon mixture according to the invention starting from an $H_2/CO$ mixture with an $H_2/CO$ molar ratio of less than 1.0 use is preferably made of an $H_2/CO$ mixture which has been prepared by steam gasification of coal at elevated temperature.

The aromatization process according to the invention is also very suitable for the preparation of p-xylene from lower hydrocarbons such as propane, propylene, butanes, butenes, n-hexane, cyclopentane and methyl cyclopentane.

In addition to being suitable as catalysts or catalyst carriers, the silicates according to the invention are also suitable for a variety of other applications, for example as adsorption and extraction agent, desiccant, ion exchanger and molecular sieve. An important application of this type is, for example, the separation of p-xylene from mixtures of p-xylene with o-xylene and m-xylene.

A number of processes which can be carried out using a silicate according to the invention are summarized below :

1. A process for the preparation and separation of p-xylene from a mixture predominantly consisting of aromatic hydrocarbons having 8 carbon atoms per molecule, in which the mixture is separated by adsorption into a mixture of p-xylene and ethyl benzene and a mixture of o-xylene and m-xylene, the p-xylene being separated by crystallization from the mixture of p-xylene and ethyl benzene, the mixture of o-xylene and m-xylene being isomerized, a further quantity of p-xylene being separated by adsorption from the isomerized product and a silicate according to the invention being used in both adsorption treatments as adsorbent.

2. A process for the separation of p-xylene from a mixture predominantly consisting of aromatic hydrocarbons having 8 carbon atoms per molecule, in which process a mixture of p-xylene and ethyl benzene is separated from the mixture by adsorption, the p-xylene being separated by crystallization from the mixture of p-xylene and ethyl benzene, use being made in the adsorption treatment of a silicate according to the invention as adsorbent, which silicate has been submitted to a pre-treatment comprising the contacting of the silicate with a solution of a salt of a polyvalent cation followed by calcination of the silicate and in which the polyvalent cation has such a charge density and the solution such a concentration of the polyvalent cation that the product of charge density and concentration is at least 45 nm. gion.$l^{-1}$.

3. A process for the preparation of liquid hydrocarbons from coal, in which

a) the coal is converted by gasification into a mixture of carbon monoxide and hydrogen,

b) the mixture of carbon monoxide and hydrogen is converted into an aromatic hydrocarbon mixture using a catalyst according to the invention,

c) an isobutene-containing gaseous fraction and an aromatic liquid fraction boiling in the gasoline range are separated from the aromatic hydrocarbon mixture,

d) the isobutane-containing gaseous fraction is converted by alkylation into a product from which a fraction boiling in the gasoline range is separated and

e) the two fractions boiling in the gasoline range obtained in accordance with c) and d) are mixed.

4. A process for the preparation of an aromatic hydrocarbon mixture in which a mixture of aliphatic oxygen-containing hydrocarbons having the general formula $C_nH_mO_p$ a predominant molar quantity of which mixture comprises one or more compounds for which

$$\frac{m-2p}{n}$$

is larger than 1 and for the rest one or more compounds for which

$$\frac{m - 2p}{n}$$

is at most 1 is contacted at elevated temperature with a catalyst according to the invention.

5. A process for the preparation of an aromatic hydrocarbon mixture from natural gas in which

a) the natural gas is converted into synthesis gas,

b) the synthesis gas is converted into an aromatic hydrocarbon mixture using a catalyst according to the invention,

c) a $C_2^-$ fraction, an isobutane-containing gaseous fraction and an aromatic liquid fraction boiling in the gasoline range are separated from the aromatic hydrocarbon mixture,

d) the $C_2^-$ fraction is recirculated to stage a) of the process,

e) the isobutane-containing gaseous fraction is converted by alkylation into a product from which a fraction boiling in the gasoline range is separated and

f) the two fractions boiling in the gasoline range obtained in accordance with c) and e) are mixed.

6. A process for the preparation of an aromatic hydrocarbon mixture from methanol, which process is carried out in two stages, dimethyl ether being prepared in the first stage by contacting methanol at elevated temperature with a dehydration catalyst and an aromatic hydrocarbon mixture being prepared in the second stage by contacting dimethyl ether from the first stage at elevated temperature with a catalyst according to the invention.

7. A process for improving the quality of a product obtained in the Fischer-Tropsch hydrocarbon synthesis process in which process a light fraction is separated from the product, which fraction predominantly comprises components boiling in and/or below the gasoline range and/or a heavy fraction predominantly comprising components boiling above the gasoline range and in which an aromatic automotive gasoline is prepared from the light fraction and/or a fuel having a low pour point from the heavy fraction by contacting the relevant fraction at elevated temperature with a catalyst according to the invention.

8. A process for the preparation of an aromatic-rich hydrocarbon mixture from an aromatic-lean hydrocarbon mixture boiling in the gasoline range in which the aromatic-lean hydrocarbon mixture is catalytically reformed and in which at least part of the reformate is contacted at elevated temperature with a catalyst according to the invention.

9. A process for the preparation of gasoline in which a hydrocarbon mixture boiling above the gasoline range is cracked using a catalyst mixture contains the components A and B, in which a fraction boiling in the gasoline range is separated from the cracked product, the catalyst component A being a crystalline aluminium silicate zeolite having a pore diameter in excess of 9 Å and catalyst component B being a silicate according to the invention.

10. A process for the preparation of a hydrocarbon mixture boiling in the gasoline range and ethylene, in which

a) a mixture of carbon monoxide and hydrogen is converted into an aromatic hydrocarbon mixture using a catalyst according to the invention,

b) a liquid fraction boiling in the gasoline range and the gaseous fraction are separated from the crude reaction product and

c) the gaseous fraction is converted by pyrolysis into a product containing ethylene.

11. A process for the preparation of a hydrocarbon mixture boiling in the gasoline range in which

a) a mixture of carbon monoxide and hydrogen is converted into an aromatic hydrocarbon mixture using a catalyst according to the invention,

b) a propane and/or butane-containing gaseous fraction and a liquid fraction boiling in the gasoline range are separated from the aromatic hydrocarbon mixture,

c) the gaseous fraction is subjected to partial dehydrogenation or partial oxidation,

d) the resultant olefinic or oxygen-containing product is converted into an aromatic hydrocarbon mixture using a catalyst according to the invention and

e) a fraction boiling in the gasoline range is separated from the aromatic hydrocarbon mixture obtained in accordance with d).

12. A process for the preparation of a gaseous fuel having a calorific value of at least 30 mega Joule/$m^3$, in which process

a) a mixture of carbon monoxide and hydrogen having an $H_2/CO$ molar ratio of less than 1 is converted into a hydrocarbon-containing reaction mixture by contacting the $H_2/CO$ mixture with a mixture of two catalysts, one of which contains zinc and/or copper and is capable of catalysing the conversion of an $H_2/CO$ mixture into acyclic, oxygen-containing hydrocarbons, and the other is a catalyst according to the invention,

b) the $C_4^-$ fraction is separated from the hydrocarbon-containing reaction mixture,

c) the gaseous fuel is prepared from the $C_4^-$ fraction by removal of at least part of the $CO_2$ present therein and

d) the conditions for the preparation of the hydrocarbon-containing reaction mixture are selected in such a way that the conversion of the $H_2/CO$ mixture is at least 70 %v.

13. A process for the preparation of an aromatic hydrocarbon mixture in which a mixture of carbon

monoxide and hydrogen having an $H_2/CO$ molar ratio between 0.25 and 0.75 is contacted with a mixture of two catalysts one of which is capable of catalysing the conversion of an $H_2/CO$ mixture into acyclic, oxygen-containing hydrocarbons and the other is a catalyst according to the invention and in which a quantity of water is added to the $H_2/CO$ mixture which quantity, in mol % based on the $H_2/CO$ mixture, is at least 2.5 and at most

$$\frac{3(V-R)}{(1+R)(1+V)}$$

in which R designates the $H_2/CO$ molar ratio of the feed and V represents the consumption ratio of the $H_2/CO$ mixture obtained under the conditions under which the above-mentioned process is carried out, but without the addition of water.

The invention will now be elucidated with reference to the following Example.

## Example

5 crystalline silicates (silicates A-E) were prepared by heating mixtures of $SiO_2$, NaOH, $[(C_3H_7)N_4]OH$ and either $Rh(NO_3)_3$ or $Cr(NO_3)_3$ or $Sc_2O_3$ in water in an autoclave under autogenous pressure at 150 °C for 24 hours. After cooling of the reaction mixtures the resultant silicates were removed by filtration, washed with water until the pH of the washing water was approximately 8 and dried at 120 °C for 2 hours. After calcining in air for one hour at 500 °C the silicates A-E had the following properties :

a) thermally stable up to a temperature in excess of 800 °C,
b) an X-ray powder diffraction pattern chiefly corresponding with that mentioned in Table B,
c) the composition expressed in moles of the oxides is as follows

silicate A 0.008 16 $M_2O \cdot 0.008\ 6\ Rh_2O_3 \cdot SiO_2$
silicate B 0.003 3 $M_2O \cdot 0.003\ 23\ Rh_2O_3 \cdot SiO_2$
silicate C 0.001 82 $M_2O \cdot 0.001\ 82\ Rh_2O_3 \cdot SiO_2$
silicate D 0.014 3 $M_2O \cdot 0.014\ 3\ Cr_2O_3 \cdot SiO_2$
silicate E 0.015 2 $M_2O \cdot 0.015\ 2\ Sc_2O_3 \cdot SiO_2$

in which M = H and Na.

The molar composition of the aqueous mixtures from which the silicates A-E were prepared are given in Table C.

TABLE C

| Silicate no. | A | B | C | D | E |
|---|---|---|---|---|---|
| $Na_2O$ | 8 | 16 | 24 | 24 | 24 |
| $Rh_2O_3$ | 1 | 1 | 1 | — | — |
| $Cr_2O_3$ | — | — | — | 1 | — |
| $Sc_2O_3$ | — | — | — | — | 1 |
| $[(C_3H_7)_4N]_2O$ | 36 | 72 | 108 | 36 | 36 |
| $SiO_2$ | 200 | 400 | 600 | 200 | 200 |
| $H_2O$ | 3 600 | 7 200 | 10 800 | 3 600 | 3 600 |

From the silicates A, D and E the silicates I-III were prepared by boiling the materials calcined at 500 °C with 1.0 molar $NH_4NO_3$ solution, washing with water, reboiling with 1.0 molar $NH_4NO_3$ solution and washing, drying at 120 °C and calcining at 500 °C.

Three catalysts (1-3) were prepared by mixing a $ZnO—Cr_2O_3$ composition with the silicates I-III. The atomic Zn percentage of the $ZnO—Cr_2O_3$ composition, based on the sum total of Zn and Cr, was 70 %. All catalyst mixtures contained 10 parts by weight of the $ZnO—Cr_2O_3$ composition per part by weight of silicate.

The catalyst mixtures 1-3 were tested for the preparation in one stage of an aromatic hydrocarbon mixture from an $H_2/CO$ mixture.

The experiment was carried out in a 50 ml reactor which contained a fixed catalyst bed with a volume of 7.5 ml. An $H_2/CO$ mixture with an $H_2/CO$ molar ratio of 0.5 was passed over the catalyst for 48 hours at a temperature of 375 °C, a pressure of 60 bar and a liquid hourly space velocity of 1 000 $Nl.l^{-}$. $hour^{-1}$.

The results of these experiments are given in Table D, wherein Experiment No.2 is a comparative example.

(See Table D page 10)

Table D

| Experiment no. | 1 | 2 | 3 |
|---|---|---|---|
| Catalyst mixture no. | 1 | 2 | 3 |
| Conversion of $H_2$/CO mixture, %v | 55 | 33 | 50 |
| Selectivity on $C_1^+$, %w | | | |
| $C_3^+$ | 85 | 86 | 91 |
| $C_5^+$ | 70 | 70 | 70 |
| Average composition of the $C_5^+$ product, %w | | | |
| Acyclic hydrocarbons | 27 | 24 | 31 |
| Naphthenes | 28 | 28 | 24 |
| Aromatics | 45 | 48 | 45 |

**Claims**

1. Catalytically active crystalline silicates having an X-ray powder diffraction pattern containing inter alia the reflections mentioned in Table A.

Table A

| Radiation : Cu-Kα | Wave length 0.154 18 nm |
|---|---|
| 2θ | relative intensity |
| 7.8-8.2 | S |
| 8.7-9.1 | S |
| 11.8-12.1 | W |
| 12.4-12.7 | W |
| 14.6-14.9 | W |
| 15.4-15.7 | W |
| 15.8-16.1 | W |
| 17.6-17.9 | W |
| 19.2-19.5 | W |
| 20.2-20.6 | W |
| 20.7-21.1 | W |
| 23.1-23.4 | VS |
| 23.8-24.1 | VS |
| 24.2-24.8 | M |
| 29.7-30.1 | M |

in which the letters used have the following significance :

VS = very strong ; S = strong, M = moderate ; W = weak ; θ = the Bragg angle, characterized in that after calcination in air for one hour the composition expressed in moles of the oxides is as follows :

$$p(0.9 \pm 0.2)M_{2/n}O \cdot p(aX_2O_3.bY_2O_3) \cdot SiO_2$$

wherein

M = H and alkali metal and/or alkaline earth metal,
X = rhodium and/or scandium,
Y = aluminium, iron and/or gallium,
$a > 0.5$,
$b \geqslant 0$,
$a + b = 1$,
$0 < p < 0.1$ and
n = the valency of M.

2. Crystalline silicates as claimed in Claim 1, characterized in that they only contain one element X and one element Y.

3. Crystalline silicates as claimed in Claim 1, characterized in that they only contain one element X and no element Y.

4. Crystalline silicates as claimed in any one of Claims 1-3, characterized in that p in the general formula is larger than 0.001 and smaller than 0.05.

5. A process for the preparation of crystalline silicates as claimed in any one of Claims 1-4, characterized in that an aqueous mixture comprising the following components :

one or more compounds containing an organic cation (R) or from which such a cation is formed during the preparation of the silicate,

one or more silicon compounds,

one or more compounds in which an element X occurs in the trivalent state and optionally

one or more compounds in which aluminium or gallium occurs or in which iron occurs in the trivalent state,

in which mixture the various compounds are present in the following ratio, expressed in moles of the oxides :

$$M_{2/n}O : R_{2/q}O = 0.1\text{-}20,$$
$$R_{2/q}O : SiO_2 = 0{\cdot}01\text{-}0.5,$$
$$SiO_2 : (X_2O_3 + Y_2O_3) > 10, \text{ and}$$
$$H_2O : SiO_2 = 5\text{-}50$$

(n and q are the valencies of M and R), is kept at elevated temperature until the silicate has formed and that the silicate is subsequently isolated from the mother liquor.

6. A process as claimed in Claim 5, characterized in that the mixture is kept at a temperature between 90 and 300 °C for at least four hours.

7. A process as claimed in any one of Claims 5 and 6, characterized in that in the aqueous mixture M is present in a sodium compound and R in a tetrapropylammonium compound.

8. A process for carrying out catalytic conversions, characterized in that use is made of a catalyst comprising a crystalline silicate as claimed in any one of Claims 1-4.

9. A process as claimed in Claim 8, characterized in that aromatic hydrocarbons are prepared from acyclic organic compounds or from synthesis gas.

**Patentansprüche**

1. Katalytisch aktive kristalline Silikate mit einem Röntgen-Pulverdiagramm, welches unter anderem die in Tabelle A angegebenen Reflexionen aufweist.

Tabelle A

| Strahlung : Cu-Kα<br>2 θ | Wellenlänge : 0,154 18 nm<br>Relative Intensität |
|---|---|
| 7,8-8,2 | S |
| 8,7-9,1 | S |
| 11,8-12,1 | W |
| 12,4-12,7 | W |
| 14,6-14,9 | W |
| 15,4-15,7 | W |
| 15,8-16,1 | W |
| 17,6-17,9 | W |
| 19,2-19,5 | W |
| 20,2-20,6 | W |
| 20,7-21,1 | W |
| 23,1-23,4 | VS |
| 23,8-24,1 | VS |
| 24,2-24,8 | M |
| 29,7-30,1 | M |

in der die verwendeten Buchstaben die folgende Bedeutung haben :

VS = sehr stark, S = stark, M = mittel ; W = schwach, θ = Braggscher Winkel, dadurch gekennzeichnet, daß nach einstündiger Calcinierung an Luft die Zusammensetzung, ausgedrückt in Mol der Oxide, folgende ist :

$$p(0{,}9 \pm 0{,}2)M_{2/n}O \cdot p(aX_2O_3.bY_2O_3) \cdot SiO_2$$

in der

M = Wasserstoff sowie Alkalimetall und/oder Erdalkalimetall,

X = Rhodium und/oder Scandium,

Y = Aluminium, Eisen und/oder Gallium,

a > 0,5,

$b \geq 0$,
$a + b = 1$,
$0 < p < 0{,}1$ und
n die Wertigkeit von M besitzt.

2. Kristalline Silikate, wie in Anspruch 1 beansprucht, dadurch gekennzeichnet, daß sie nur ein Element X und ein Element Y enthalten.

3. Kristalline Silikate, wie in Anspruch 1 beansprucht, dadurch gekennzeichnet, daß die nur ein Element X und kein Element Y enthalten.

4. Kristalline Silikate, wie in jedem einzelnen der Ansprüche 1 bis 3 beansprucht, dadurch gekennzeichnet, daß p in der allgemeinen Formel größer als 0,001 und Kleiner als 0,05 ist.

5. Ein Verfahren Zur Herstellung kristaliner Silikate, wie in jedem einzelnen der Ansprüche 1 bis 4 beansprucht, dadurch gekennzeichnet, daß ein wässriges Gemisch, welches die folgenden Bestandteile aufweist :

eine oder mehrereVerbindung(en), die ein organisches Kation(R) enthält (enthalten) oder aus der (denen) ein solches Kation während der Herstellung des Silikats gebildet wird,

eine oder mehrere Siliciumverbindung(en),

eine oder mehrere Verbindung(en), in denen ein Element X im dreiwertigen Zustand vorliegt, sowie gegebenenfalls,

eine oder mehrere Verbindung(en), in der (denen) Aluminium oder Gallium vorliegt, oder in der (denen) Eisen im dreiwertigen Zustand vorliegt,

wobei in dem Gemisch die verschiedenen Verbindungen im folgenden Verhältnis, ausgedrückt in Mol der Oxide, vorliegen :

$$M_{2/n}O : R_{2/q}O = 0{,}1 \text{ bis } 20$$
$$R_{2/q}O : SiO_2 = 0{,}01 \text{ bis } 0{,}5$$
$$SiO_2 : (X_2O_3 + Y_2O_3) > 10 \text{ und}$$
$$H_2O : SiO_2 = 5 \text{ bis } 50$$

(n und q sind die Wertigkeiten von M und R)
auf erhöhter Temperatur gehalten wird, bis sich das Silikat gebildet hat, und daß das Silikat anschließend von der Mutterlauge abgetrennt wird.

6. Ein Verfahren, wie in Anspruch 5 beansprucht, dadurch gekennzeichnet, daß das Gemisch mindestens vier Stunden lange auf einer Temperatur zwischen 90 und 300 °C gehalten wird.

7. Ein Verfahren, wie in irgendeinem der Ansprüche 5 und 6 beansprucht, dadurch gekennzeichnet, daß in dem wässrigen Gemisch M in einer Natriumverbindung und R in einer Tetrapropylammoniumbindung vorliegt.

8. Ein Verfahren zur Durchführung katalytischer Umwandlungen, dadurch gekennzeichnet, daß ein Katalysator verwendet wird, welcher ein wie in jedem einzelnen der Ansprüche 1 bis 4 beanspruchtes kristallines Silikat aufweist.

9. Ein Verfahren wie in Anspruch 8 beansprucht, dadurch gekennzeichnet, daß aromatische Kohlenwasserstoffe aus acyclischen organischen Verbindungen oder aus Synthesegas hergestellt werden.

## Revendications

1. Silicates cristallins catalytiquement actifs ayant un diagramme de diffraction des rayons X par la méthode des poudres ayant, entre autres, les réflexions mentionnées dans le Tableau A :

Tableau A

| Radiation : Cu-Kα | Longueur d'onde 0,154 18 nm |
|---|---|
| 2 θ | intensité relative |
| 7,8-8,2 | S |
| 8,7-9,1 | S |
| 11,8-12,1 | W |
| 12,4-12,7 | W |
| 14,6-14,9 | W |
| 15,4-15,7 | W |
| 15,8-16,1 | W |
| 17,6-17,9 | W |
| 19,2-19,5 | W |
| 20,2-20,6 | W |
| 20,7-21,1 | W |
| 23,1-23,4 | VS |

| | |
|---|---|
| 23,8-24,1 | VS |
| 24,2-24,8 | M |
| 29,7-30,1 | M |

où les lettres utilisées ont la signification suivante :

VS = très forte ; S = forte ; M = modérée ; W = faible ; $\theta$ = l'angle de Bragg, caractérisés en ce qu'après calcination dans l'air pendant une heure, la composition exprimée en moles des oxydes est la suivante :

$$p(0,9 \pm 0,2)M_{2/n}O \cdot p(aX_2O_3 \cdot bY_2O_3) \cdot SiO_2$$

où

M = H, un métal alcalin et/ou un métal alcalino-terreux,
X = rhodium et/ou scandium,
Y = aluminium, fer et/ou gallium,
$a > 0,5$,
$b \geqslant 0$,
$a + b = 1$,
$0 < p < 0,1$ et
m = la valence de M.

2. Silicates cristallins selon la revendication 1, caractérisés en ce qu'ils contiennent un élément X et un élément Y.

3. Silicates cristallins selon la revendication 1, caractérisés en ce qu'ils contiennent seulement un élément X et pas d'élément Y.

4. Silicates cristallins selon l'une quelconque des revendications 1 à 3, caractérisés en ce que p dans la formule générale est plus grand que 0,001 et plus petit que 0,05.

5. Procédé pour la préparation de silicates cristallins tels que revendiqués dans l'une quelconque des revendications 1 à 4, caractérisé en ce qu'un mélange aqueux comprenant les constituants suivants :

un ou plusieurs composés contenant un cation organique (R) ou à partir desquels un tel cation est formé durant la préparation du silicate,

un ou plusieurs composés du silicium,

un ou plusieurs composés dans lesquels un élément X est présent à l'état trivalent et éventuellement,

un ou plusieurs composés dans lesquels de l'aluminium ou du gallium est présent ou dans lesquels du fer est présent à l'état trivalent,

mélange dans lequel les divers composés sont présents dans les rapports suivants, exprimés en moles des oxydes :

$$M_{2/n}O : R_{2/q}O = 0,1\text{-}20$$
$$R_{2/q}O : SiO_2 = 0,01\text{-}0,5$$
$$SiO_2 : (X_2O_3 + Y_2O_3) > 10 \text{ et}$$
$$H_2O : SiO_2 = 5\text{-}20$$

(n et q sont les valences de M et R), est maintenu à température élevée jusqu'à ce que le silicate soit formé, et que le silicate est ensuite insolé à partir de la liqueur-mère.

6. Procédé selon la revendication 5, caractérisé en ce que le mélange est maintenu à une température comprise entre 90 et 300 °C pendant au moins quatre heures.

7. Procédé selon l'une quelconque des revendications 5 et 6, caractérisé en ce que dans le mélange aqueux M est présent dans un composé du sodium et R dans un composé de tétrapropylammonium.

8. Procédé pour effectuer des conversions catalytiques, caractérisé en ce qu'on utilise un catalyseur comprenant un silicate cristallin selon l'une quelconque des revendications 1 à 4.

9. Procédé selon la revendication 8, caractérisé en ce que des hydrocarbures aromatiques sont préparés à partir de composés organiques acycliques ou à partie de gaz de synthèse.